# FASCICULE DE BREVET EUROPEEN

(11) **EP 4 228 712 B1**
(45) Date de publication et mention de la délivrance du brevet: **27.11.2024**
(21) Numéro de dépôt: 21807174.4
(22) Date de dépôt: 13.10.2021
(51) Int. Cl.: A61L 9/20, E04B 9/00, E04B 9/02, E04B 9/30

(54) **STRUCTURE DE PLAFOND ET INSTALLATION COMPRENANT UNE TELLE STRUCTURE DE PLAFOND COMPRENANT DES MOYENS DE STERILISATION DE L'AIR**
DECKENSTRUKTUR UND ANLAGE MIT EINER SOLCHEN DECKENSTRUKTUR MIT LUFTSTERILISATIONSMITTELN
CEILING STRUCTURE AND FACILITY COMPRISING SUCH A CEILING STRUCTURE COMPRISING AIR STERILISATION MEANS

(30) Priorité: 13.10.2020 FR 2010441
(43) Date de publication de la demande: 23.08.2023
(73) Titulaire: Scherrer, Jean-Marc, 68400 Riedisheim (FR); Lang, Damien, 67100 Strasbourg (FR)
(72) Inventeur: Scherrer, Jean-Marc, 68400 Riedisheim (FR); Lang, Damien, 67100 Strasbourg (FR)
(74) Mandataire: IP Trust
(86) Numéro de dépôt international: PCT/FR2021/051777
(87) Numéro de publication internationale: WO 2022/079388

(56) Documents cités:
- CN-A- 105 756 256
- FR-A1- 3 085 696
- KR-A- 20190 061 183
- KR-Y1- 200 362 941
- US-A1- 2019 292 315

## Description

### DOMAINE TECHNIQUE DE L'INVENTION

La présente invention concerne le domaine des plafonds amenant un air, traité ou non, dans les locaux associés. Par air traité, on entend un air chauffé, refroidi, ventilé, déshumidifié ou humidifié.

L'invention concerne plus particulièrement une structure de plafond et une installation comprenant une telle structure de plafond et permettant d'amener un air traité dans un local, comprenant une paroi de plafond, et une fausse paroi positionnée en sous face et à distance de tout ou partie de la paroi de plafond et délimitant, avec la paroi de plafond ou une portion de la paroi de plafond, un couloir de circulation d'air comprenant une ouverture de sortie d'air à l'intérieur du local ménagée dans la fausse paroi.

La structure de plafond et l'installation associée selon l'invention sont destinées notamment mais non exclusivement à la stérilisation de l'air délivré dans un local.

### ETAT DE LA TECHNIQUE

On connait des exemples de réalisations de structures de plafond permettant de délivrer un air traité, et notamment stérilisé par ultraviolet, à l'intérieur du local associé.

Il peut être cité la demande WO2020/050864 A1 (SYNERGY MED GLOBAL DESIGN SOLUTIONS LLC A DELAWARE LLC [US]) laquelle divulgue également une installation de plafond dans une salle d'opération et destinée à réduire le risque d'infections de patient (HAI), comprenant des structures raccordées au plafond qui permettent de délivrer à l'intérieur du local des rideaux d'air stérilisé à écoulement laminaire. Dans cette installation, la stérilisation de l'air est réalisée dans la partie technique de ventilation avant son injection directement à l'intérieur du local. Intégrés aux moyens assurant la ventilation, l'accès aux moyens intervenant dans la stérilisation de l'air est rendue difficile, voire délicate. Par ailleurs, la qualité de stérilisation de l'air dépend de la durée d'exposition de ce dernier au sein de la centrale de ventilation. Pour augmenter la durée d'exposition, il est nécessaire de diminuer le débit aéraulique de la centrale ou de disposer de sections de passage de l'air très importantes de sorte à en diminuer sa vitesse. L'obtention d'une stérilisation d'air efficace est donc particulièrement difficile et coûteuse.

Il peut être également cité la demande de brevet GB2127954 (WEISS TECH UMWELT KLIMA) laquelle divulgue un plafond d'alimentation en air pour une salle d'opération. Le plafond d'alimentation en air aspire l'air d'alimentation par une ouverture ménagée dans la paroi latérale du local, située au-dessus d'un plafond suspendu de section. La section centrale du plafond d'alimentation en air comprend un conduit d'air dont l'entrée s'ouvre à l'intérieur du local et qui est alimenté en air par un ventilateur. L'entrée d'air est pourvue d'un filtre stérilisant au travers duquel l'air est soufflé.

Le plafond d'alimentation en air de la demande GB2127954 présente l'inconvénient de n'assurer qu'une stérilisation de l'air limitée du fait d'un traitement uniquement lors de sa traversée du filtre, en entrée du conduit d'air. La stérilisation de l'air n'est assurée que sur une fraction de temps correspondant au temps de traversée du filtre par l'air. Aussi, dans ce plafond, la qualité de la stérilisation de l'air est fonction du débit de l'air traversant le filtre.

Ce plafond d'alimentation en air présente également l'inconvénient d'imposer l'emplacement du système de traitement (conduit d'air et filtre) à celui des ouvertures ménagées dans le faux plafond.

Par ailleurs, compte tenu du positionnement du système de traitement sur le faux-plafond ainsi que de son encombrement, l'installation décrite ne peut être étendue à des structures de plafonds mettant en oeuvre des toiles tendues ou des systèmes couvrant la surface du plafond.

Le document FR 3 085 696 A1 (EGIS BATIMENTS [FR]; NORMALE [FR]) divulgue un plafond technique rayonnant permettant le chauffage, la climatisation et la ventilation d'un local, caractérisé en ce qu'il comprend au moins une toile tendue laissant passer en tout ou partie la lumière et/ou le rayonnement thermique, ladite toile (3) s'étendant entre des parois et étant arrangée pour définir avec une dalle supérieure un espace, et un ou plusieurs éléments rayonnants, formés par exemple de panneaux métalliques, et arrangés pour permettre la circulation d'un fluide caloporteur/frigoporteur, les éléments rayonnants étant disposés dans l'espace ménagé entre la toile tendue (3) et la dalle supérieure. Le plafond technique, selon sa mise en oeuvre, peut être un faux plafond ou un îlot autonome.

Le document KR 200 362 941 Y1 divulgue un dispositif d'éclairage d'ascenseur, le vent provenant du trou de ventilation (20) traverse le mécanisme de guidage de ventilation (30) et est soufflé dans l'ascenseur, ce dispositif d'éclairage (40) comprend une lampe de stérilisation à ultraviolets.

Le document KR 2019 0061183 A (SILVER ELEVATOR KOREA CO LTD [KR]) décrit un ascenseur impliquant un stérilisateur d'air utilisant des rayons ultraviolets (UV) et du plasma. Plus précisément, il est proposé un ascenseur stérilisant pour hôpital afin de prévenir des infections secondaires dans les hôpitaux, l'une des principales sources de virus et de bactéries. Pour surmonter cette pollution, il est fourni un système d'ascenseur propre afin de gérer la qualité de l'air grâce à une stérilisation efficace de l'espace. La technologie UV haute puissance est appliquée. De plus, grâce à la prévention fondamentale des bactéries infectieuses, les gènes sociales et pertes économiques peuvent être prévenues à l'avance en évitant la propagation d'une infection secondaire dans l'hôpital.

Le document CN 105 756 256 B (ZHEJIANG FASHION BUILDING MAT CO LTD) décrit un plafond intégré à fonction désinfectante. Le plafond intégré comprend un boîtier, dans lequel un mécanisme d'éclairage, un mécanisme de ventilation, un mécanisme de chauffage, un mécanisme de désinfection et un contrôleur sont disposés dans le boîtier ; le mécanisme d'éclairage comprend une pluralité de tubes de lampe d'éclairage disposés en parallèle, un abat-jour transparent est disposé sur la surface inférieure du boîtier et la pluralité de tubes de lampe d'éclairage est située sur le côté intérieur de l'abat-jour transparent ; le mécanisme de désinfection comprend une pluralité de tubes de lampe à ultraviolets disposés en parallèle, des volets sont disposés sur la surface inférieure du boîtier, sont reliés à un moteur d'entraînement et comprennent une pluralité de lames disposées de manière épissée, les surfaces des lames sont des surfaces de miroir , une pluralité de trous traversant sont formés uniformément dans chaque lame, et la pluralité de tubes de lampe ultraviolette sont situés sur les côtés intérieurs des obturateurs ; le mécanisme de ventilation comprend un canal de soufflage d'air, deux extrémités du canal de soufflage d'air sont reliées en correspondance un à un avec un tube d'entrée d'air et un tube de sortie d'air, le mécanisme de réchauffement est connecté en série au tube de sortie d'air, l'ouverture d'extrémité externe du tube de sortie d'air forme une fenêtre d'échappement, la pluralité de tubes de lampe à ultraviolets sont tous situés dans la fenêtre d'échappement, et un soufflage d'air est disposé dans le canal de soufflage d'air.

Le document US 2019/292315 A1 (NIEMIEC DARRIN [US]; CARLSON WILLIAM [US]) décrit un ventilateur axial combiné et un système d'éclairage à LED configurés pour s'adapter à l'empreinte d'une dalle de plafond standard. Des modes de réalisation décrits comprennent en outre des dalles de plafond avec un ventilateur intégré et/ou un éclairage LED. Les systèmes divulgués peuvent comprendre un conteneur de logement et un ventilateur axial. Le ventilateur a une cavité de ventilateur comprenant un mécanisme de dérivation d'air pour diriger l'air de la cavité de ventilateur vers les composants d'éclairage et de ventilateur. L'invention comprend une surface de circulation d'air pour diriger l'air existant dans la cavité du ventilateur le long d'un luminaire à LED. De plus, les modes de réalisation décrits comprennent une ou plusieurs sources de lumière UV qui irradient des contaminants lorsque l'air s'écoule à travers la dalle de plafond. Le système d'aspiration de l'air d'un local à traite se fait de manière verticale au niveau du ventilateur, des déflecteurs vont orienter l'air aspiré à stériliser de manière multidirectionnelle vers des chambres latérales ou logements où un système de réflexion des rayons UV est disposé sur toute la surface du logement afin d'améliorer l'action des lampes ultraviolettes.

L'invention vise à remédier à ces problèmes en proposant une structure de plafond permettant d'amener dans un local un air stérilisé, dont la qualité de stérilisation est garantie et améliorée par rapport à celle proposée par les structures de plafond de l'état de la technique.

La structure de plafond a également pour but d'assurer une stérilisation de l'air contrôlée et régulière, dont l'efficacité est beaucoup moins liée au débit de l'air à traiter.

La structure de plafond a également pour but d'être mise en oeuvre avec un faux-plafond, avantageusement à toile tendue, sans nuire à l'esthétisme de ce dernier.

### BREVE DESCRIPTION DE L'INVENTION

A cet effet, l'invention propose un local selon la revendication 1.

Le local à stériliser comprend un faux plafond avec:
- au moins une toile tendue de protection, étanche aux UVC, ladite toile tendue de protection positionnée en sous face et à distance du plafond du local à stériliser, étant fixée aux parois latérales dudit local et arrangée avec une dalle supérieure du plafond pour délimiter un espace dit plénum,
- au moins un moyen d'accroche de ladite au moins une toile tendue de protection aux parois latérales du local au moyen d'au moins un profilé d'accroche périphériques comportant une fente de sortie d'air donnant sur au moins une paroi latérale du local et permettant le passage de l'air circulant entre le plénum et ledit local à stériliser,
- au moins un moyen de ventilation et/ou climatisation connecté audit plénum par l'intermédiaire d'au moins un conduit de ventilation aéraulique d'entrée d'air (9) dans ledit plénum, l'air soufflé formant un couloir unidirectionnel horizontal de circulation d'air sur toute la longueur du plénum,
- un caisson de retour d'air apte à être connecté à des moyens de ventilation et/ou climatisation, ainsi que des ouvertures d'aspiration d'air situées à l'opposé de ladite fente de sortie d'air et permettant le passage de l'air depuis l'intérieur du local vers ledit caisson de retour d'air, et où ledit faux plafond comporte une pluralité de sources lumineuses UVC disposée dans le plénum sur toute la longueur dudit couloir unidirectionnel horizontal de circulation d'air de sorte à stériliser l'air s'écoulant dans ledit couloir de circulation d'air avant son passage au travers de la fente de sortie d'air et où la distance entre ladite pluralité de sources lumineuses UVC et le plafond d'une part et la distance entre ladite pluralité de sources lumineuses UVC et ladite au moins une toile tendue de protection d'autre part est au maximum de 25 cm.

En particulier, la présente invention se propose de fournir un faux plafond permettant d'amener un air traité ou non dans un local à stériliser, comprenant :
- au moins une toile tendue de protection, étanche aux UVC, ladite toile tendue de protection positionnée en sous face et à distance du plafond du local à stériliser, étant fixée aux parois latérales dudit local et arrangée avec une dalle supérieure du plafond pour délimiter un espace dit plénum,
- au moins une toile tendue de finition, ladite toile tendue, visible depuis le local, étant fixée aux parois latérales dudit local,
- au moins un moyen d'accroche de ladite au moins une toile tendue de protection et de ladite au moins une toile tendue de finition aux parois latérales du local au moyen d'au moins un profilé d'accroche périphériques comportant une fente de sortie d'air donnant sur au moins une paroi latérale du local et permettant le passage de l'air circulant entre le plénum et ledit local à stériliser,
- au moins un moyen de ventilation et/ou climatisation connecté audit plénum par l'intermédiaire d'au moins un conduit de ventilation aéraulique d'entrée d'air dans ledit plénum, l'air soufflé formant un couloir unidirectionnel horizontal de circulation d'air sur toute la longueur du plénum,
- un caisson de retour d'air apte à être connecté à des moyens de ventilation et/ou climatisation, ainsi que des ouvertures d'aspiration d'air situées à l'opposé de ladite fente de sortie d'air et permettant le passage de l'air depuis l'intérieur du local vers ledit caisson de retour d'air, et où ledit faux plafond comporte une pluralité de sources lumineuses UVC disposée dans le plénum sur toute la longueur dudit couloir unidirectionnel horizontal de circulation d'air de sorte à stériliser l'air s'écoulant dans ledit couloir de circulation d'air avant son passage au travers de la fente de sortie d'air et où la distance entre ladite pluralité de sources lumineuses UVC et le plafond d'une part et la distance entre ladite pluralité de sources lumineuses UVC et ladite au moins une toile tendue de protection d'autre part est au maximum de 25 cm.

Selon l'invention, la pluralité de sources lumineuses émet des UVC.

Avantageusement, les moyens lumineux comportent une pluralité de sources lumineuses réparties sur tout ou partie de la paroi ou de la portion de paroi du plafond.

Avantageusement, la pluralité des sources lumineuses sont disposées à mi-hauteur du plénum.

Avantageusement, la fausse paroi est une fausse paroi à toile tendue. Dans ce cas, et avantageusement, la structure de plafond comporte des moyens bloquant la lumière ultraviolette émise par la pluralité des sources lumineuses de sorte à former un écran de protection de la toile tendue de la fausse paroi. Selon une configuration préférée, les moyens bloquant la lumière ultraviolette comportent une toile tendue additionnelle disposée entre la toile tendue de la fausse paroi et la paroi de plafond.

Avantageusement, la structure de plafond comporte en outre un caisson de retour d'air apte à être raccordé ou connecté fluidiquement à des moyens de ventilation et/ou climatisation, ainsi que des ouvertures d'aspiration d'air permettant le passage de l'air depuis l'intérieur du local dans le caisson. Ainsi, outre l'insufflation d'un air stérilisé à l'intérieur du local, la structure de plafond assure le retour d'air insufflé vers des moyens de ventilation et/ou climatisation auxquels le caisson de retour d'air sera raccordé.

Selon un mode de réalisation avantageux, les moyens de ventilation et/ou de climatisation sont raccordé(s) également fluidiquement au caisson de retour d'air.

### BREVE DESCRIPTION DES FIGURES

D'autres caractéristiques et avantages de l'invention ressortiront de la description détaillée de l'invention qui va suivre en référence aux figures annexées et dans lesquelles :
[Fig. 1] La **figure 1** représente une coupe transversale d'une structure de faux plafond permettant la circulation d'un air ambiant traité au sein d'un local selon un exemple de réalisation de l'invention pour lequel les sources lumineuses sont installées contre le plafond de la pièce.
[Fig. 2] La **figure 2** représente une coupe transversale d'une structure de faux plafond permettant la circulation d'un air ambiant traité au sein d'un local selon un autre exemple de réalisation de l'invention pour lequel les sources lumineuses sont installées à mi-hauteur du plénum.
[Fig. 3] La **figure 3** représente une vue du haut à savoir depuis le plafond d'une structure de faux plafond permettant la circulation d'un air ambiant traité au sein d'un local selon un autre exemple de réalisation de l'invention pour lequel les sources lumineuses sont installées en lignes continues ou discontinues.
[Fig. 4] La **figure 4** représente une vue du haut à savoir depuis le plafond d'une structure de faux plafond permettant la circulation d'un air ambiant traité au sein d'un local selon un autre exemple de réalisation de l'invention pour lequel les sources lumineuses sont installées en lignes discontinues et où deux lignes de lampes lumineuses discontinues sont mitoyennes l'agencement de leurs sources lumineuses UVC est disposé en quinconce.
[Fig. 5] La **figure 5** présente une coupe horizontale issue du résultat des simulations numériques.
[Fig. 6] La **figure 6** présente l'impact de la hauteur du plénum (4) sur la qualité de désinfection.
[Fig. 7] La **figure 7** représente le taux de désinfection en % de l'air intérieur de la pièce ou local à traiter en fonction du temps de fonctionnement des sources lumineuses.

### DESCRIPTION DETAILLEE DE L'INVENTION

Bien que des méthodes et des matériaux similaires ou équivalents à ceux décrits ici puissent être utilisés dans la pratique, des méthodes et des matériaux appropriés sont décrits ci-dessous. Toutes les publications, demandes de brevet, brevets et autres références mentionnées ici sont incorporées par référence dans leur intégralité. De plus, les matériaux, les méthodes et les modes de réalisations décrits sont uniquement illustratifs et ne sont pas destinés à être limitatifs.

En cas de conflit, la présente description, y compris les définitions, prévaudra.

Sauf définition contraire, tous les termes techniques et scientifiques utilisés dans le présent document ont la même signification que celle généralement comprise par l'homme du métier auquel le sujet appartient. Telles qu'utilisées ici, les définitions suivantes sont fournies afin de faciliter la compréhension de la présente invention.

Le terme «comprendre ou comprend» est généralement utilisé dans le sens d'inclure, c'est-à-dire de permettre la présence d'une ou plusieurs caractéristiques ou composants.

Tel qu'utilisé dans la description et les revendications, les formes singulières « un », « une » et « le », « la » incluent des références plurielles, sauf indication contraire du contexte.

Le terme « air traité » signifie un air refroidi ou réchauffé (de sorte à assurer les besoins de refroidissement ou de chauffage d'un local) et doté d'un apport d'air neuf extérieur de sorte à assurer la ventilation d'air hygiénique du local.

En règle générale, il est entendu par « air froid » une température allant jusqu'à 20°C et « air chaud » toute température située au-delà de 20°C.

La présente invention se propose de fournir un faux plafond (1) permettant d'amener un air traité ou non dans un local à stériliser, comprenant :
- au moins une toile tendue de protection (8), étanche aux UVC, ladite toile tendue de protection (8) positionnée en sous face et à distance du plafond (3) du local à stériliser, étant fixée aux parois latérales dudit local et arrangée avec une dalle supérieure du plafond (3) pour délimiter un espace dit plénum (4),
- au moins un moyen d'accroche de ladite au moins une toile tendue de protection (8) aux parois latérales du local au moyen d'au moins un profilé d'accroche périphériques (12) comportant une fente de sortie d'air (5) donnant sur au moins une paroi latérale du local et permettant le passage de l'air circulant entre le plénum (4) et ledit local à stériliser,
- au moins un moyen de ventilation et/ou climatisation (10) connecté audit plénum (4) par l'intermédiaire d'au moins un conduit de ventilation aéraulique d'entrée d'air (9) dans ledit plénum (4), l'air soufflé formant un couloir unidirectionnel horizontal de circulation d'air sur toute la longueur du plénum (4),
- un caisson (15) de retour d'air apte à être connecté à des moyens de ventilation et/ou climatisation (10), ainsi que des ouvertures d'aspiration d'air (14) situées à l'opposé de ladite fente de sortie d'air (5) et permettant le passage de l'air depuis l'intérieur du local vers ledit caisson de retour d'air (15),
et où ledit faux plafond (1) comporte une pluralité de sources lumineuses UVC (6) disposée dans le plénum (4) sur toute la longueur dudit couloir unidirectionnel horizontal de circulation d'air de sorte à stériliser l'air s'écoulant dans ledit couloir de circulation d'air avant son passage au travers de la fente de sortie d'air (5) et où la distance entre ladite pluralité de sources lumineuses UVC (6) et le plafond (3) d'une part et la distance entre ladite pluralité de sources lumineuses UVC (6) et ladite au moins une toile tendue de protection (8) d'autre part est au maximum de 25 cm.

De préférence, le faux plafond (1) selon l'invention comprend en outre au moins une toile tendue de finition (2), ladite toile tendue de finition (2), visible depuis le local, étant fixée aux parois latérales dudit local au moyen d'au moins un profilé d'accroche périphériques (12) comportant une fente de sortie d'air (5) donnant sur au moins une paroi latérale du local et permettant le passage de l'air circulant entre le plénum (4) et ledit local à stériliser.

Aussi, selon un mode de réalisation préféré, l'invention se propose de fournir un faux plafond (1) permettant d'amener un air traité ou non dans un local à stériliser, comprenant :
- au moins une toile tendue de protection (8), étanche aux UVC, ladite toile tendue de protection (8) positionnée en sous face et à distance du plafond (3) du local à stériliser, étant fixée aux parois latérales dudit local et arrangée avec une dalle supérieure du plafond (3) pour délimiter un espace dit plénum (4),
- au moins une toile tendue de finition (2), ladite toile tendue (2), visible depuis le local, étant fixée aux parois latérales dudit local,
- au moins un moyen d'accroche de ladite au moins une toile tendue de protection (8) et de ladite au moins une toile tendue de finition (2) aux parois latérales du local au moyen d'au moins un profilé d'accroche périphériques (12) comportant une fente de sortie d'air (5) donnant sur au moins une paroi latérale du local et permettant le passage de l'air circulant entre le plénum (4) et ledit local à stériliser,
- au moins un moyen de ventilation et/ou climatisation (10) connecté audit plénum (4) par l'intermédiaire d'au moins un conduit de ventilation aéraulique d'entrée d'air (9) dans ledit plénum (4), l'air soufflé formant un couloir unidirectionnel horizontal de circulation d'air sur toute la longueur du plénum (4),
- un caisson (15) de retour d'air apte à être connecté à des moyens de ventilation et/ou climatisation (10), ainsi que des ouvertures d'aspiration d'air (14) situées à l'opposé de ladite fente de sortie d'air (5) et permettant le passage de l'air depuis l'intérieur du local vers ledit caisson de retour d'air (15),
et où ledit faux plafond (1) comporte une pluralité de sources lumineuses UVC (6) disposée dans le plénum (4) sur toute la longueur dudit couloir unidirectionnel horizontal de circulation d'air de sorte à stériliser l'air s'écoulant dans ledit couloir de circulation d'air avant son passage au travers de la fente de sortie d'air (5) et où la distance entre ladite pluralité de sources lumineuses UVC (6) et le plafond (3) d'une part et la distance entre ladite pluralité de sources lumineuses UVC (6) et ladite au moins une toile tendue de protection (8) d'autre part est au maximum de 25 cm.

De préférence, la hauteur du plénum (4) est au maximum de 50 cm.

Selon un mode de réalisation particulier, la hauteur du plénum (4) est d'au moins 10 cm.

Avantageusement, ladite pluralité de sources lumineuses UVC (6) est disposée à mi-hauteur du plénum (4).

Selon un mode de réalisation, ladite pluralité de sources lumineuses UVC (6) est agencée sous la forme de lignes de lampes lumineuses (13) qui sont espacées sur toute la longueur dudit plénum (4).

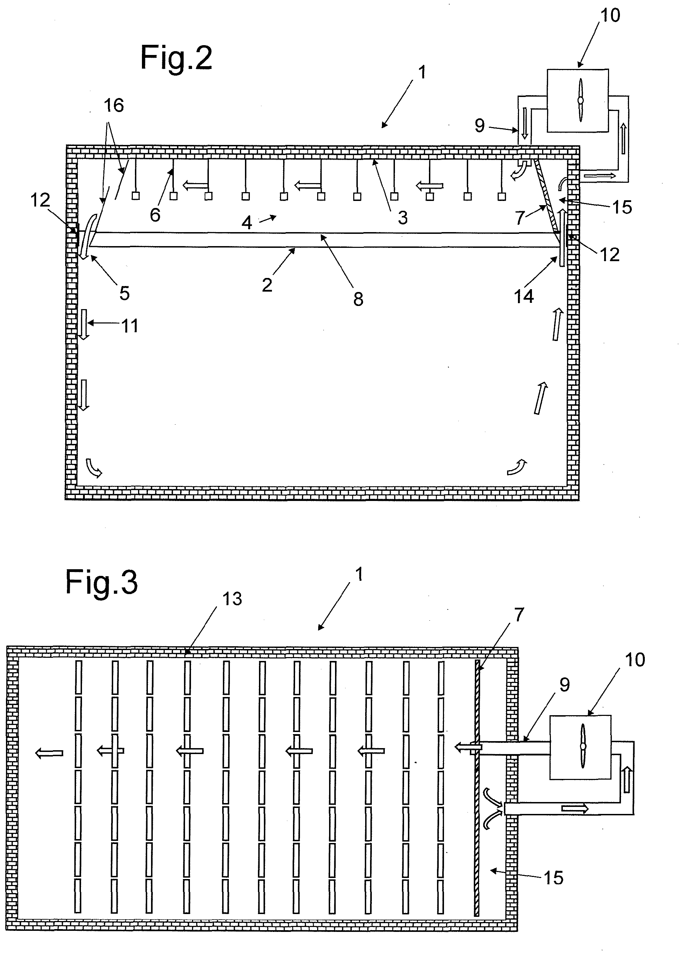

Selon un autre mode de réalisation de l'invention, lesdites lignes de lampes lumineuses (13) sont des lignes continues ou discontinues (voir figure 3).

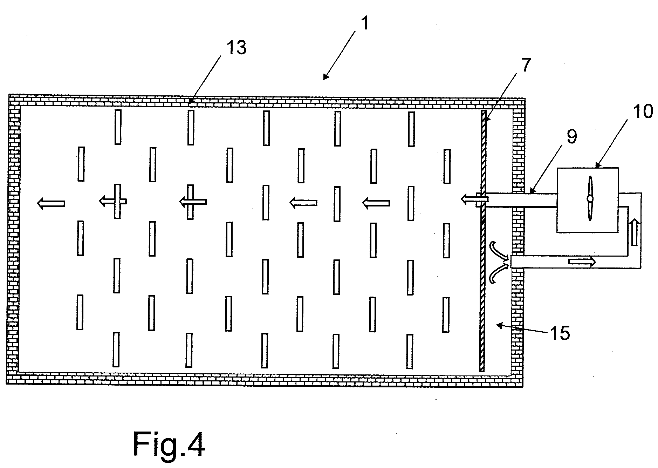

De préférence dans le cas où deux lignes de lampes lumineuses (13) discontinues sont mitoyennes l'agencement de leurs sources lumineuses UVC (6) est disposé en quinconce (voir figure 4). Par quinconce, il est entendu que les sources lumineuses UVC (6) de deux lignes de lampes lumineuses (13) mitoyennes vont alternent sur deux rangées.

Avantageusement, l'espace entre deux lignes de lampes lumineuses (13) est compris entre 25 cm et 200 cm.

De préférence, l'espace entre deux lignes de lampes lumineuses (13) est compris entre 50 cm et 100 cm.

De manière surprenante, il a été démontré que l'air dudit local à stériliser présente un coefficient de désinfection de 99.9% des virus et bactéries lorsque ledit moyen de ventilation et/ou climatisation (10) est activé pendant au plus 30 minutes et ce indépendamment du nombre d'occupant dans ledit local à stériliser.

Par ailleurs il a aussi été démontré que l'air dudit local à stériliser présente un coefficient de désinfection de 99.9% des virus et bactéries lorsque ledit moyen de ventilation et/ou climatisation (10) est activé pendant au moins 8 minutes et ce indépendamment du nombre d'occupant dans ledit local à stériliser.

De préférence, les parois du plénum (4) comportent des moyens de réflexion de la lumière ultraviolette émise par ladite pluralité de sources lumineuses UVC (6).

Selon un mode préféré de l'invention, la face de la toile tendue de protection (8) donnant sur le plénum (4) possède un coefficient de réflexion aux UVC supérieur ou égal à 80%.

Avantageusement, ladite toile tendue de protection (8) et ladite toile de de finition (2) ont une épaisseur de moins de 5 mm.

Selon un autre mode de réalisation, ledit canal d'aspiration (15) est formé par une cloison de séparation (7) solidaire dudit profilé d'accroche périphérique (12).

L'invention se propose également de fournir une installation permettant une amenée d'air traité dans un local à stériliser, comprenant :
- un faux plafond (1) selon l'invention,
- une entrée d'air (9) dans le plénum (4), la fente de sortie d'air (5) de la fausse paroi étant ménagée en extrémité opposée de l'entrée d'air (9), et
- des moyens de ventilation et/ou de climatisation (10) connectés à l'entrée d'air (9) afin de former un couloir unidirectionnel horizontal de circulation d'air du plénum (4).

Avantageusement, les moyens de ventilation et/ou de climatisation (10) sont connectés au caisson (15) de retour d'air.

### DESCRIPTION DETAILLEE DES FIGURES

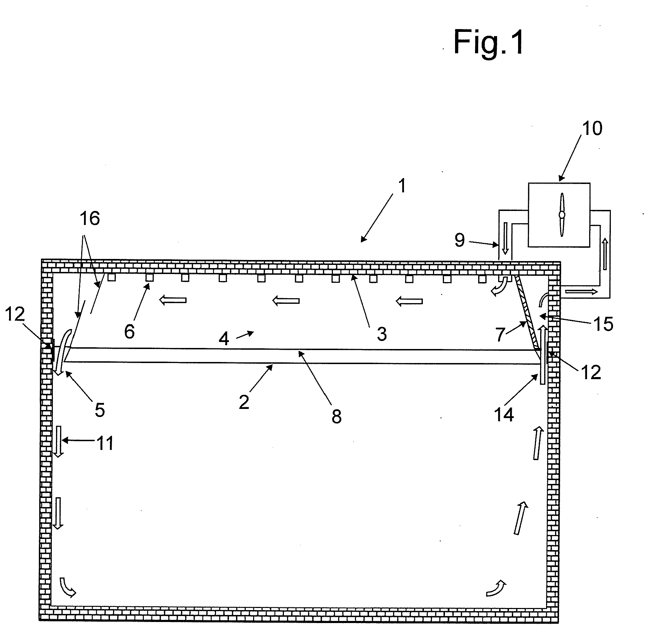

En relation avec la **figure 1****,** il est décrit une structure de plafond ou faux plafond (1) permettant l'amenée d'un air traité (11) au sein d'un local. Dans la présente invention, le traitement concerne la stérilisation de l'air.

Le faux plafond (1) comprend dans l'exemple illustré une toile tendue de protection (8), positionnée en sous face de la paroi de plafond (3). La toile tendue de protection (8) s'étend entre les parois murales du local à stériliser et est fixée à ces parois par l'intermédiaire de profilés d'accroche (12) (appelés également lisses). On désignera dans ce qui suit la fausse paroi à toile tendue par première toile de protection (8) afin de simplifier la lecture de la présente description.

La première toile tendue (8) forme une toile pouvant être visible depuis l'intérieur du local. Elle est arrangée pour délimiter avec la paroi de plafond (3) un couloir de circulation d'air (4) (également appelé plénum).

Le faux plafond (1) comprend une entrée d'air ou ventilation aéraulique ( 9) dans le couloir de circulation d'air ou plénum (4) et une ouverture ou fente de sortie d'air (5) à l'intérieur du local à traiter. Un système de ventilation et/ou climatisation (10), raccordé au conduit (9) d'entrée d'air, permet d'assurer dans tout le volume du couloir de circulation d'air (4), un écoulement d'air laminaire entre l'entrée d'air (9) et la fente de sortie d'air (5). L'ouverture de sortie d'air (5) est ménagée dans le profilé d'accroche périphérique (12), ce dernier formant un profilé dit d'amenée d'air. La demande WO 2018/037184, au nom du Demandeur, décrit un exemple d'un tel profilé pouvant être mis en oeuvre avec la structure de faux plafond (1) selon l'objet de la présente invention.

Selon l'invention, le faux plafond (1) comporte une pluralité de moyens lumineux (6) qui sont, dans l'exemple représenté, fixés sur la dalle ou paroi de plafond (3) ou suspendus dans le plénum (4).

Dans le mode de réalisation illustré, la pluralité de moyens lumineux (6) consistent en des sources lumineuses UVC (6) réparties sur la paroi de plafond (3) ou suspendues à la paroi de plafond (3), et aptes à émettre de la lumière ultraviolette. Préférentiellement, ces sources lumineuses UVC (6) sont des lampes, par exemple des diodes électroluminescentes, de type UVC. Ces sources, ayant une fonction germicide, permettent ainsi la stérilisation de l'air lors de son passage dans le couloir de circulation d'air (4).

La première toile de protection (8), est traitée avec par exemple une composition permettant d'empêcher le passage des UVC. Elle permet de protéger ainsi les personnes présentes dans le local en bloquant les UVC.

Avantageusement, un déflecteur (16) est positionné au droit ou au niveau de la fente de sortie d'air (5) de sorte à interdire tout passage des UVC par cette dernière et ainsi assurer une protection parfaite des occupants du local.

Avantageusement, le faux plafond (1) comporte des moyens bloquant la lumière ultraviolette émise par les sources lumineuses UVC (6), ceci afin de former un écran de protection de la toile tendue de protection (8). Cet écran de protection est réalisé dans l'exemple illustré par une toile tendue de finition (2) additionnelle (désigné de deuxième toile tendue 2) visible depuis le local et disposée en contrebas de la première toile tendue (8). Comme illustrée, la deuxième toile tendue (2) est disposée à distance de la première toile tendue (8) et est visible depuis le local. Dans le cas où le faux plafond (1) comporte une telle toile de finition (2), l'entrée d'air débouche dans le couloir de circulation d'air (4), entre la première toile tendue (8) et la paroi de plafond (3). Il est bien entendu prévu une entrée d'air dans l'espace délimité par la deuxième toile tendue (2) et la première toile tendue (8). Dans ce cas, des sources lumineuses pourront être prévues sur la paroi murale dans laquelle l'entrée d'air est ménagée et sur la paroi murale au voisinage de la fente de sortie d'air (5) vers l'intérieur du local. Comme précédemment, les ouvertures de sortie d'air (5) sont ménagées tant pour la première toile tendue (8) que pour la deuxième toile tendue (2), au niveau des profilés de lisse (12) sur laquelle sont fixées les deux toiles tendues. La présence de la deuxième toile tendue (2) est bien entendu facultative, et il peut être prévu un faux plafond (1) sans toile additionnelle (2). De même, il peut être prévu un faux plafond (1) comprenant plusieurs toiles de protection additionnelles, de manière avantageuse disposées parallèles entre elles et à distance les unes des autres de manière à définir une pluralité de couloirs de circulation d'air. Dans ce cas, chaque profilé de lisse ou d'accroche périphérique (12) comprenant une ouverture de sortie d'air (5) dans le couloir sous-jacent attenant. Les profilés de lisse (12) présentent ainsi des ouvertures de sortie d'air (5) arrangées les unes par rapport aux autres pour définir une circulation descendante sensiblement verticale le long de la paroi murale, jusqu'à la traversée de l'ouverture de sortie d'air (5) débouchant dans le local. Par ailleurs, dans le mode de réalisation illustré, les deux toiles sont accrochées par l'intermédiaire de profilés de lisse (12) communs. Il peut bien entendu être prévu un profilé de lisses distincts pour chacune des toiles sans sortir du cadre de l'invention

Comme illustré, l'air provenant des ouvertures d'aspiration d'air (14) est soufflé par le système de ventilation ou climatisation (10) dans le couloir de circulation ou plénum (4) par l'intermédiaire du conduit d'entrée d'air (9). Dès son entrée dans le couloir de circulation d'air (4), le débit d'air est soumis à une section de passage beaucoup plus importante que celle du conduit d'amenée d'air (9), la vitesse d'air diminue alors fortement engendrant un flux d'air laminaire entrainé suivant une trajectoire sensiblement parallèle à la paroi de plafond (3), sous les sources lumineuses UVC (6). L'air est ainsi soumis aux rayonnements des sources lumineuses UVC (6) sur toute la longueur du couloir de circulation et pendant une durée importante. A titre d'exemple, pour une pièce de 20m² (5m x 4m) alimentée par 800m³/h d'air traité et pour un couloir de circulation d'air de 30cm de hauteur, l'air passera d'une vitesse conventionnelle d'environ 2,5 m/s dans le conduit (9) à0,18 m/s dès son entrée dans le couloir de circulation d'air (4), soit une réduction d'un facteur 14. Ainsi, dans cette configuration, la durée minimale d'exposition d'une molécule d'air sera de 28 secondes. A titre comparatif elle serait de 0,4 secondes pour un dispositif de stérilisation inséré sur 1m du conduit d'amenée d'air (9). Cet arrangement présente l'avantage de garantir le traitement de l'air, quel que soit son débit. En effet, suivant la valeur du débit d'air et la géométrie de la pièce à traiter, il est possible de déterminer la hauteur du couloir de circulation d'air ou plénum (4) de sorte à obtenir le temps d'exposition aux UVC souhaité. L'air traverse ensuite l'ouverture de sortie de la première toile (8) puis l'ouverture de sortie de la deuxième toile (2) de sorte à être diffusé vers la pièce ou local à traiter.

Dans le mode de réalisation représenté, la structure de plafond ou faux plafond (1) comprend un plafond tendu. L'invention ne se limite pas à une telle structure de plafond, les structures comprenant des dalles de plafond suspendues, des plafonds métalliques ou plâtre, des îlots suspendus ou tout autre type de plafond rentrant dans le champ d'application de l'invention.

Outre la stérilisation de l'air traversant le couloir de circulation d'air (4), l'invention présente l'avantage d'assurer une stérilisation de tous les corps solides donnant sur le couloir de circulation (4) comme la face inférieure du plafond (3), les retombées verticales entre le plafond (3) et la première toile (8) ou encore tout élément qui serait installé au sein du couloir de circulation (4).

Avantageusement les surfaces du plénum constituées par le couloir de circulation d'air (4) comme la sous face du plafond (3) ou alors les retombées verticales entre le plafond (3) et la première toile (8) peuvent être habillée d'un matériau hygiénique comme une tôle d'acier inoxydable ou un revêtement polymère (comme le PVC par exemple) notamment pour les usages dans des locaux contraints à de fortes contraintes hygiéniques comme les hôpitaux.

Avantageusement le matériau hygiénique utilisé pour habiller les surfaces de plénum (4) est réfléchissant évitant ainsi toute perte du rayonnement UVC émis au sein du plénum par absorption du matériau hygiénique.

Dans un mode de réalisation particulier, comme illustré sur les **figures 1** à **4****,** la structure de plafond comprend à la fois le soufflage d'air traité et stérilisé vers la pièce ou local à traiter et l'aspiration d'air intérieur vers l'unité de traitement d'air. Dans ce cas de figure l'aspiration d'air est réalisée le long d'une paroi opposée à la paroi de soufflage d'air via une ouverture d'aspiration d'air (14) équipée d'un caisson d'un retour d'air (15).

L'invention est décrite dans ce qui précède à titre d'exemple. Il est entendu que l'homme du métier est à même de réaliser différentes variantes de réalisation de l'invention sans pour autant sortir du cadre de l'invention.

### Exemples :

### Exemple 1 :

Des calculs d'efficacité prévisionnelle de désinfection ont été menés via des simulations numériques (CFD) sur la base d'une pièce définie de 4m de largeur par 5m de longueur avec 2,5m de hauteur sous plafond, c'est-à-dire sous la toile tendue de finition (2).

La hauteur du plénum (4) est de 20cm entre le plafond naturel de la pièce (3) et la toile tendue de protection (8).

D'autres simulations ont été menées en faisant varier la hauteur du plénum (4) jusqu'à une hauteur totale de 1m, de sorte à évaluer l'impact de ce paramètre sur la qualité de désinfection.

L'unité de climatisation / ventilation (10) assure un débit d'air de 343m³/h, correspondant à une puissance thermique de 70W/m² au sol, puissance couramment utilisée pour les besoin des refroidissement des bâtiments en France.

L'aspiration d'air est réalisée le long d'une paroi de 4m via une ouverture d'aspiration (14) de 24mm une gaine d'aspiration rectangulaire est connectée à un caisson d'aspiration (15) composé d'une cloison de séparation (7) soutenue par le profilé d'accroche périphérique (12).

Le soufflage d'air traité par l'unité de climatisation (10) dans le plénum (4) par le réseau rectangulaire aussi appelé ventilation aéraulique d'entrée d'air (9) via 6 orifices rectangulaires de 300mm x 100mm répartis sur la largeur de la pièce.

La fente de soufflage d'air (5) vers la pièce, de 24mm de largeur, est située sur la petite longueur de 4m à l'opposé de l'ouverture d'aspiration d'air (14).

Des lampes UVC (6) de marque OSRAM HNS 8W G5 de type tube cylindrique (diamètre 16mm et longueur 288mm) sont disposées en quinconce suivant des lignes (13) à mi-hauteur du plénum (4), espacement de 50cm entre chaque ligne de lampes (13) soit 9 lignes de lampes UVC réparties sur la longueur de 5m, 7 lampes UVC (6) par ligne (13).

Une autre configuration a été testée comprenant 3 lignes (13) de lampes UVC (6) soit une ligne tous les 1,25m.

Chaque lampe UVC (6) dispose d'une puissance unitaire d'émission UVC de 2W selon les données du fabricant.

Les seuils de désinfection d'air utilisés, également appelés dosages, pour les simulations, c'est-à-dire l'énergie que doit recevoir une molécule d'air pour être débarrassée de x % des virus et bactéries dans l'air ont été transmis par le fabricant des lampes UVC Osram. Ces seuils sont exprimés en énergie surfacique soit la puissance reçue par une molécule d'air multipliée par le temps d'exposition:
- Dosage de 2,6 mJ/cm2 pour 90% de désinfection,
- Dosage de 5,0 mJ/cm2 pour 99% de désinfection,
- Dosage de 8,8 mJ/cm2 pour 99,9% de désinfection.

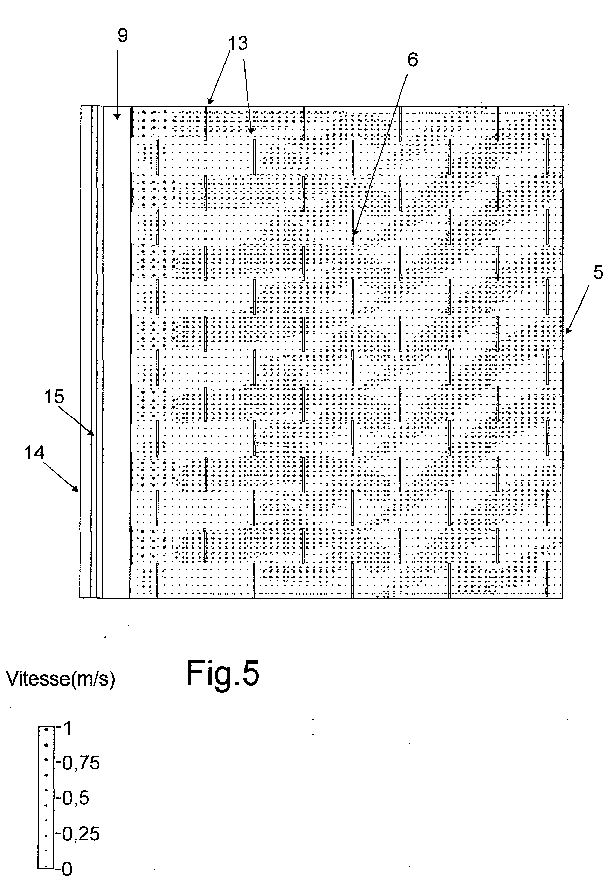

La **figure 5** présente une coupe horizontale issue du résultat des simulations numériques. Cette coupe est située à hauteur des sources lumineuses (6) installées dans le plénum (4).

La modélisation présente notamment la comportement du flux d'air soufflé dans le plénum par visualisation des vitesses d'air.

On y distingue notamment les lignes (13) de sources lumineuses UVC (6), les sources lumineuses UVC (6) disposées en quiconque, le caisson d'aspiration d'air (15), l'ouverture d'aspiration d'air (14) et la fente de soufflage d'air (5).

Le conduit de ventilation aéraulique d'entrée d'air (9) éjecte l'air soufflé dans le plénum (4) par l'intermédiaire de 6 orifices dirigés vers le côté droit du plénum.

Ce modèle qui a permis de déterminer les résultats d'efficacité décrits ci-après permet notamment de constater que le flux d'air se transforme en flux laminaire lors de son parcours dans le plénum, évoluant à des vitesses très lentes et soumis donc à une durée d'exposition importante au rayonnement UVC émis par les sources lumineuses. Ainsi la géométrie du système de faux plafond (1) selon l'invention impose un temps d'exposition beaucoup plus long et donc une efficacité de stérilisation améliorée.

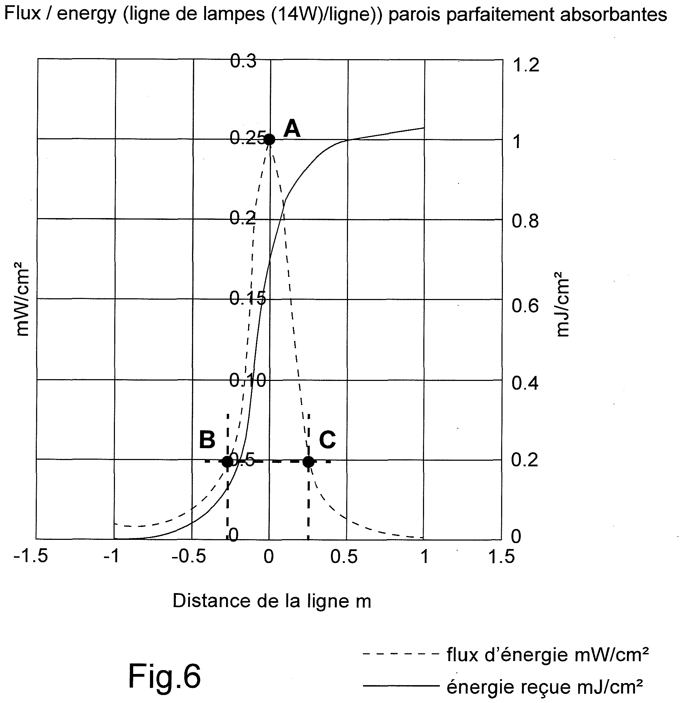

La **figure 6** présente l'impact de la hauteur du plénum (4) sur la qualité de désinfection.

En effet la qualité de désinfection est directement liée à la quantité d'énergie en mJ/m2 reçue par le flux d'air en présence dans le plénum.

Cette courbe donne en abscisse la distance entre une molécule d'air et une ligne (13) de 7 sources lumineuses UVC (6). En ordonnée on obtient la quantité d'énergie reçue par une molécule d'air, fonction de la distance entre cette molécule et la ligne de sources lumineuses.

La courbe adopte la forme d'une courbe de Gauss. Le point A représente une molécule d'air située contre la ligne (13) de sources lumineuses UVC.

Le point B représente une molécule d'air située 25cm sous la ligne (13) de sources lumineuses UVC.

Le point C représente une molécule d'air située 25cm au-dessus de la ligne (13) de sources lumineuses UVC (6).

Le résultat des simulations donne une quantité d'énergie de précisément 1,02mJ/m2 reçue par le point A.

Ainsi, plus on s'éloigne des sources lumineuses (6) plus la quantité d'énergie reçue par l'air et donc la qualité de désinfection diminue.

On constate notamment que si l'on souhaite conserver une partie raisonnable (20%) de l'énergie émise par la ligne (13) de sources lumineuses, l'air ne doit pas être en mesure de passer à plus de 25cm en dessous ou au-dessus de la ligne (13) des sources lumineuses UVC (points B et C) ce qui correspond, avec la ligne de sources lumineuses UVC idéalement placée à mi-hauteur du plénum (4), à une hauteur de plénum maximale de 50cm.

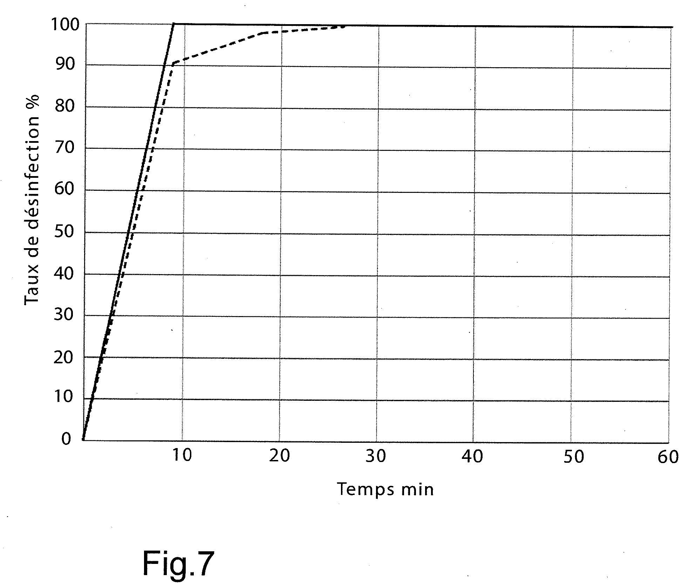

La **figure 7** représente le taux de désinfection en % de l'air intérieur de la pièce ou local à traiter en fonction du temps de fonctionnement des sources lumineuses (6) et donc du système.

2 configurations ont été calculées :
9 lignes (13) de sources lumineuses espacées de 50cm pour la courbe continue,
3 lignes (13) de sources lumineuses espacées de 1,25m pour la courbe en pointillés.

Les calculs ont été réalisés pour une hauteur de plénum (4) de 20cm.

On y observe que pour 9 lignes de sources lumineuses (6), moins de 8 à 9min de fonctionnement du système de ventilation (10) sont nécessaires à l'atteinte d'un taux de désinfection de 99,9% de l'air intérieur de la pièce et que pour 3 lignes de sources lumineuses (6), moins de 27min de fonctionnement du système de ventilation (10) sont nécessaires à l'atteinte d'un taux de désinfection de 99,9% de l'air intérieur de la pièce ceci indépendamment du nombre d'occupants de la pièce.

En conclusion, le système ainsi testé présente de par la géométrie du plénum (4) un écoulement laminaire du flux d'air au sein de ce dernier présentant l'avantage d'une longue durée d'exposition au flux UVC émis par la pluralité des sources lumineuses (6).

La hauteur du plénum limitée à 50cm permet de bénéficier d'au moins 20% du rayonnement UVC émis par les sources lumineuses (6) permettant de limiter le nombre et la puissance de ces dernières.

Avec un espacement entre les lignes (13) de sources lumineuses UVC (6) compris entre 50cm et 1,25m, un temps réduit de fonctionnement du système (entre 8-9 et 27 minutes) permet l'atteinte d'un taux de désinfection de l'air intérieur de la pièce de 99,9%.

### Numéros de références employés dans les figures:

1 : Faux plafond
2 : toile tendue de finition
3 : plafond du local à stériliser
4 : espace dit plénum
5 : fente de sortie d'air
6 : pluralité de sources lumineuses UVC
7 : cloison de séparation
8 : toile tendue de protection
9 : ventilation aéraulique d'entrée d'air
10 : moyens de ventilation et/ou climatisation
11 : amenée d'un air traité
12 : profilé d'accroche périphériques
13 :lignes de lampes lumineuses
14 : ouvertures d'aspiration d'air
15 : canal d'aspiration d'air aussi dénommé caisson de retour d'air
16 : déflecteur.

## Revendications

1. Local à stériliser comprenant :
- un faux plafond (1) permettant d'amener un air traité ou non dans ledit local à stériliser, comprenant :
au moins une toile tendue de protection (8), étanche aux UVC, ladite toile tendue de protection (8) positionnée en sous face et à distance du plafond (3) du local à stériliser, étant fixée aux parois latérales dudit local et arrangée avec une dalle supérieure du plafond (3) pour délimiter un espace dit plénum (4),
au moins un moyen d'accroche de ladite au moins une toile tendue de protection (8) aux parois latérales du local au moyen d'au moins un profilé d'accroche périphériques (12) comportant une fente de sortie d'air (5) donnant sur au moins une paroi latérale du local et permettant le passage de l'air circulant entre le plénum (4) et ledit local à stériliser,
- au moins un moyen de ventilation et/ou climatisation (10) connecté audit plénum (4) par l'intermédiaire d'au moins un conduit de ventilation aéraulique d'entrée d'air (9) dans ledit plénum (4), l'air soufflé formant un couloir unidirectionnel horizontal de circulation d'air sur toute la longueur du plénum (4),
- un caisson (15) de retour d'air apte à être connecté à des moyens de ventilation et/ou climatisation (10), ainsi que des ouvertures d'aspiration d'air (14) situées à l'opposé de ladite fente de sortie d'air (5) et permettant le passage de l'air depuis l'intérieur du local vers ledit caisson de retour d'air (15),
et où ledit faux plafond (1) comporte une pluralité de sources lumineuses (6) disposée dans le plénum (4) sur toute la longueur dudit couloir unidirectionnel horizontal de circulation d'air et où la distance entre ladite pluralité de sources lumineuses UVC (6) et le plafond (3) d'une part et la distance entre ladite pluralité de sources lumineuses UVC (6) et ladite au moins une toile tendue de protection (8) d'autre part est au maximum de 25 cm,
**caractérisé en ce que** ladite pluralité de sources lumineuses (6) sont des sources UVC de sorte à stériliser l'air s'écoulant dans le couloir de circulation d'air avant son passage au travers de la fente de sortie d'air (5) et **en ce que** les parois du plénum (4) comportent des moyens de réflexion de la lumière ultraviolette émise par ladite pluralité de sources lumineuses UVC (6).

2. Local à stériliser selon la revendication 1, **caractérisé en ce que** le faux plafond (1) comprend en outre au moins une toile tendue de finition (2), ladite toile tendue de finition (2), visible depuis le local, étant fixée aux parois latérales dudit local au moyen d'au moins un profilé d'accroche périphériques (12) comportant une fente de sortie d'air (5) donnant sur au moins une paroi latérale du local et permettant le passage de l'air circulant entre le plénum (4) et ledit local à stériliser.

3. Local à stériliser selon la revendication 1 ou 2, **caractérisé en ce que** la hauteur du plénum (4) est au maximum de 50 cm.

4. Local à stériliser selon l'une quelconque des revendications 1 à 3, **caractérisé en ce que** la hauteur du plénum (4) est d'au moins 10 cm.

5. Local à stériliser selon l'une quelconque des revendications 1 à 4, **caractérisé en ce que** ladite pluralité de sources lumineuses UVC (6) est disposée à mi-hauteur du plénum (4).

6. Local à stériliser selon l'une quelconque des revendications 1 à 5, **caractérisé en ce que** ladite pluralité de sources lumineuses UVC (6) est agencée sous la forme de lignes de lampes lumineuses (13) qui sont espacées sur toute la longueur dudit plénum (4).

7. Local à stériliser selon l'une quelconque des revendications 1 à 6, **caractérisé en ce que** lesdites lignes de lampes lumineuses (13) sont des lignes continues ou discontinues.

8. Local à stériliser selon la revendication 7, **caractérisé en ce que** dans le cas où deux lignes de lampes lumineuses (13) discontinues sont mitoyennes l'agencement de leurs sources lumineuses UVC (6) est disposé en quinconce.

9. Local à stériliser selon l'une quelconque des revendications 1 à 8, **caractérisé en ce que** l'espace entre deux lignes de lampes lumineuses (13) est compris entre 25 cm et 200 cm.

10. Local à stériliser selon l'une quelconque des revendications 1 à 9, **caractérisé en ce que** l'espace entre deux lignes de lampes lumineuses (13) est compris entre 50 cm et 100 cm.

11. Local à stériliser selon l'une quelconque des revendications 1 à 10, **caractérisé en ce que** l'air dudit local à stériliser présente un coefficient de désinfection de 99.9% des virus et bactéries lorsque ledit moyen de ventilation et/ou climatisation (10) est activé pendant au plus 30 minutes et ce indépendamment du nombre d'occupant dans ledit local à stériliser.

12. Local à stériliser selon l'une quelconque des revendications 1 à 10, **caractérisé en ce que** l'air dudit local à stériliser présente un coefficient de désinfection de 99.9% des virus et bactéries lorsque ledit moyen de ventilation et/ou climatisation (10) est activé pendant au moins 8 minutes et ce indépendamment du nombre d'occupant dans ledit local à stériliser.

13. Local à stériliser selon l'une quelconque des revendications précédentes, **caractérisé en ce que** la face de la toile tendue de protection (8) donnant sur le plénum (4) possède un coefficient de réflexion aux UVC supérieur ou égal à 80%.

14. Local à stériliser selon l'une quelconque des revendications 2 à 13, **caractérisé en ce que** ladite toile tendue de protection (8) et ladite toile de de finition (2) ont une épaisseur de moins de 5 mm.

15. Local à stériliser selon l'une quelconque des revendications précédentes, **caractérisé en ce que** ledit canal d'aspiration (15) est formé par une cloison de séparation (7) solidaire dudit profilé d'accroche périphérique (12).

## Patentansprüche

1. Zu sterilisierender Raum, umfassend:
- eine Zwischendecke (1), die es ermöglicht, aufbereitete oder nicht aufbereitete Luft in den zu sterilisierenden Raum zu leiten, umfassend:
mindestens ein gespanntes, UVC-dichtes Schutzgewebe (8), wobei das gespannte Schutzgewebe (8) an der Unterseite und im Abstand von der Decke (3) des zu sterilisierenden Raums positioniert und an den Seitenwänden des Raums befestigt ist, und mit einer oberen Deckenplatte (3) versehen ist, um einen als Plenum (4) bezeichneten Raum abzugrenzen,
mindestens ein Aufhängmittel des mindestens einen gespannten Schutzgewebes (8) an den Seitenwänden des Raums mittels mindestens eines umlaufenden Aufhängeprofils (12), das einen sich zu mindestens einer Seitenwand des Raums öffnenden Luftauslassschlitz (5) umfasst und den Durchtritt der Luft ermöglicht, die zwischen dem Plenum (4) und dem zu sterilisierenden Raum zirkuliert,
- mindestens ein Lüftungs- und/oder Klimatisierungsmittel (10), das mit dem Plenum (4) über mindestens einen lufttechnischen Lüftungskanal für den Lufteinlass (9) in das Plenum (4) verbunden ist, wobei die eingeblasene Luft einen horizontalen, unidirektionalen Luftzirkulationskanal über die gesamte Länge des Plenums (4) bildet,
- einen Luftrückführkasten (15), der an Lüftungs- und/oder Klimatisierungsmittel (10) angeschlossen werden kann, sowie Luftansaugöffnungen (14), die sich gegenüber dem Luftauslassschlitz (5) befinden und den Luftdurchtritt vom Inneren des Raums zum Luftrückführkasten (15) ermöglichen,
und wobei die Zwischendecke (1) eine Vielzahl von Lichtquellen (6) umfasst, die im Plenum (4) über die gesamte Länge des horizontalen, unidirektionalen Luftzirkulationskanals angeordnet sind, und wobei der Abstand zwischen der Vielzahl von UVC-Lichtquellen (6) und der Decke (3) einerseits und der Abstand zwischen der Vielzahl von UVC-Lichtquellen (6) und dem mindestens einen gespannten Schutzgewebe (8) andererseits maximal 25 cm beträgt,
**dadurch gekennzeichnet, dass** die Vielzahl von Lichtquellen (6) UVC-Quellen sind, um die im Luftzirkulationskanal strömende Luft vor ihrem Durchtritt durch den Luftauslassschlitz (5) zu sterilisieren, und dass die Wände des Plenums (4) Mittel zum Reflektieren des von der Vielzahl von UVC-Lichtquellen (6) emittierten ultravioletten Lichts umfassen.

2. Zu sterilisierender Raum nach Anspruch 1,
**dadurch gekennzeichnet, dass** die Zwischendecke (1) ferner mindestens ein gespanntes Abschlussgewebe (2) umfasst, wobei das gespannte Abschlussgewebe (2) vom Raum aus sichtbar an den Seitenwänden des Raums mittels mindestens eines umlaufenden Aufhängeprofils (12) befestigt ist, das einen Luftaustrittsschlitz (5) umfasst, der sich zu mindestens einer Seitenwand des Raums öffnet und den Durchtritt der zwischen dem Plenum (4) und dem zu sterilisierenden Raum zirkulierenden Luft ermöglicht.

3. Zu sterilisierender Raum nach Anspruch 1 oder 2,
**dadurch gekennzeichnet, dass** die Höhe des Plenums (4) maximal 50 cm beträgt.

4. Zu sterilisierender Raum nach einem der Ansprüche 1 bis 3,
**dadurch gekennzeichnet, dass** die Höhe des Plenums (4) mindestens 10 cm beträgt.

5. Zu sterilisierender Raum nach einem der Ansprüche 1 bis 4,
**dadurch gekennzeichnet, dass** die Vielzahl von UVC-Lichtquellen (6) auf halber Höhe des Plenums (4) angeordnet ist.

6. Zu sterilisierender Raum nach einem der Ansprüche 1 bis 5,
**dadurch gekennzeichnet, dass** die Vielzahl von UVC-Lichtquellen (6) in Form von Reihen von Leuchtstofflampen (13) angeordnet ist, die über die gesamte Länge des Plenums (4) verteilt sind.

7. Zu sterilisierender Raum nach einem der Ansprüche 1 bis 6,
**dadurch gekennzeichnet, dass** die Reihen von Leuchtstofflampen (13) durchgehende oder unterbrochene Reihen sind.

8. Zu sterilisierender Raum nach Anspruch 7,
**dadurch gekennzeichnet, dass** im Fall von zwei unterbrochenen Reihen von Leuchtstofflampen (13) nebeneinander die Anordnung ihrer UVC-Lichtquellen (6) versetzt angeordnet ist.

9. Zu sterilisierender Raum nach einem der Ansprüche 1 bis 8,
**dadurch gekennzeichnet, dass** der Abstand zwischen zwei Reihen von Leuchtstofflampen (13) zwischen 25 cm und 200 cm beträgt.

10. Zu sterilisierender Raum nach einem der Ansprüche 1 bis 9,
**dadurch gekennzeichnet, dass** der Abstand zwischen zwei Reihen von Leuchtstofflampen (13) zwischen 50 cm und 100 cm beträgt.

11. Zu sterilisierender Raum nach einem der Ansprüche 1 bis 10,
**dadurch gekennzeichnet, dass** die Luft des zu sterilisierenden Raums einen Desinfektionskoeffizienten für Viren und Bakterien von 99,9 % aufweist, wenn Lüftungs- und/oder Klimatisierungsmittel (10) für höchstens 30 Minuten und unabhängig von der Anzahl der Personen im zu sterilisierenden Raum aktiviert sind.

12. Zu sterilisierender Raum nach einem der Ansprüche 1 bis 10,
**dadurch gekennzeichnet, dass** die Luft des zu sterilisierenden Raums einen Desinfektionskoeffizienten für Viren und Bakterien von 99,9 % aufweist, wenn Lüftungs- und/oder Klimatisierungsmittel (10) mindestens 8 Minuten und unabhängig von der Anzahl der Personen im zu sterilisierenden Raum aktiviert sind.

13. Zu sterilisierender Raum nach einem der vorstehenden Ansprüche,
**dadurch gekennzeichnet, dass** die dem Plenum (4) zugewandte Seite des gespannten Schutzgewebes (8) einen UVC-Reflexionskoeffizienten von größer oder gleich 80 % aufweist.

14. Zu sterilisierender Raum nach einem der Ansprüche 2 bis 13,
**dadurch gekennzeichnet, dass** das gespannte Schutzgewebe (8) und das Abschlussgewebe (2) eine Dicke von weniger als 5 mm aufweisen.

15. Zu sterilisierender Raum nach einem der vorstehenden Ansprüche,
**dadurch gekennzeichnet, dass** der Ansaugkanal (15) durch eine Trennwand (7) gebildet wird, die mit dem umlaufenden Aufhängeprofil (12) fest verbunden ist.

## Claims

1. A room to be sterilized, comprising:
- a false ceiling (1) for supplying treated or not treated air in said room to be sterilized, comprising:
at least one protective tensioned fabric (8), blocking UVC, said protective tensioned fabric (8) positioned on the underside and at a distance from the ceiling (3) of the space to be sterilized, being fixed to the side walls of said space and arranged with an upper slab of the ceiling (3) in order to delimit a so-called plenum (4) space,
at least one means for attaching said at least one protective tensioned fabric (8) to the side walls of the space by means of at least one peripheral attachment profile (12) comprising an air outlet slot (5) giving onto at least one side wall of the space and allowing the passage of the air circulating between the plenum (4) and said space to be sterilized,
- at least one ventilation and/or air-conditioning means (10) connected to said plenum (4) via at least one air inlet air venting duct (9) in said plenum (4), the blown air forming a horizontal unidirectional air circulation corridor over the entire length of the plenum (4),
- an air return box (15) able to be connected to ventilation and/or air conditioning means (10), as well as air suction openings (14) located opposite said air outlet slot (5) and allowing air to pass from inside the space to said air return box (15),
and where said false ceiling (1) includes a plurality of light sources (6) arranged in the plenum (4) over the entire length of said horizontal unidirectional air circulation corridor and where the distance between said plurality of UVC light sources (6) and the ceiling (3) on the one hand and the distance between said plurality of UVC light sources (6) and said at least one stretched protective fabric (8) on the other hand is at most 25 cm,
**characterized in that** said plurality of light sources (6) are UVC sources so as to sterilize the air flowing in the air circulation corridor before its passage through the air outlet slot (5) and **in that** the walls of the plenum (4) comprise means for reflecting the ultraviolet light emitted by said plurality of UVC light sources (6).

2. The room to be sterilized according to claim 1, **characterized in that** the false ceiling (1) further comprises at least one tensioned finishing fabric (2), said tensioned finishing fabric (2), visible from the room, being fixed to the side walls of said room by means of at least one peripheral attachment profile (12) comprising an air outlet slot (5) giving onto at least one side wall of the room and allowing the passage of the air circulating between the plenum (4) and said room to be sterilized.

3. The room to be sterilized according to claim 1 or 2,
**characterized in that** the height of the plenum (4) is at most 50 cm.

4. The room to be sterilized according to any of claims 1 to 3,
**characterized in that** the height of the plenum (4) is at least 10 cm.

5. The room to be sterilized according to any of claims 1 to 4,
**characterized in that** said plurality of UVC light sources (6) is arranged at mid-height of the plenum (4).

6. The room to be sterilized according to any of claims 1 to 5,
**characterized in that** said plurality of UVC light sources (6) is arranged in the form of lines of light lamps (13) that are spaced apart over the entire length of said plenum (4).

7. The room to be sterilized according to any of claims 1 to 6,
**characterized in that** said lines of light lamps (13) are continuous or discontinuous lines.

8. The room to be sterilized according to claim 7, **characterized in that,** in the case where two discontinuous lines of light lamps (13) are adjoining the arrangement of their UVC light sources (6) is arranged in staggered rows.

9. The room to be sterilized according to any of claims 1 to 8,
**characterized in that** the space between two lines of light lamps (13) is between 25 cm and 200 cm.

10. The room to be sterilized according to any of claims 1 to 9,
**characterized in that** the space between two lines of light lamps (13) is between 50 cm and 100 cm.

11. The room to be sterilized according to any of claims 1 to 10,
**characterized in that** the air of said room to be sterilized has a disinfection coefficient of 99.9% of the viruses and bacteria when said ventilation and/or air conditioning means (10) is activated for at most 30 minutes and this independently of the number of occupants in said room to be sterilized.

12. The room to be sterilized according to any of claims 1 to 10,
**characterized in that** the air of said room to be sterilized has a disinfection coefficient of 99.9% of the viruses and bacteria when said ventilation and/or air conditioning means (10) is activated for at least 8 minutes and this independently of the number of occupants in said room to be sterilized.

13. The room to be sterilized according to any of the preceding claims,
**characterized in that** the face of the protective tensioned fabric (8) giving onto the plenum (4) has a UVC reflection coefficient of greater than or equal to 80%.

14. The room to be sterilized according to any of claims 2 to 13,
**characterized in that** said protective tensioned fabric (8) and said finishing fabric (2) have a thickness of less than 5 mm.

15. The room to be sterilized according to any of the preceding claims, **characterized in that** said suction channel (15) is formed by a separation partition (7) integral with said peripheral attachment profile (12).
